(19) **Europäisches Patentamt**

**European Patent Office**

**Office européen des brevets**

(11) **EP 1 565 427 B1**

(12) **EUROPEAN PATENT SPECIFICATION**

(45) Date of publication and mention
of the grant of the patent:
**17.05.2006 Bulletin 2006/20**

(51) Int Cl.:
***C07C 251/24*** *(2006.01)*

(21) Application number: **03779856.8**

(86) International application number:
**PCT/EP2003/012410**

(22) Date of filing: **03.11.2003**

(87) International publication number:
**WO 2004/046087 (03.06.2004 Gazette 2004/23)**

(54) **PROCESS FOR THE PREPARATION OF AN ENANTIOMERICALLY ENRICHED SCHIFF BASE**

VERFAHREN ZUR HERSTELLUNG EINER ENANTIOMERENANGEREICHERTEN SCHIFF BASE

PROCEDE PERMETTANT DE PREPARER UNE BASE DE SCHIFF ENANTIOMERIQUEMENT ENRICHIE

(84) Designated Contracting States:
**AT BE BG CH CY CZ DE DK EE ES FI FR GB GR HU IE IT LI LU MC NL PT RO SE SI SK TR**

(30) Priority: **15.11.2002 EP 02102596**

(43) Date of publication of application:
**24.08.2005 Bulletin 2005/34**

(73) Proprietor: **DSM IP Assets B.V.**
**6411 TE Heerlen (NL)**

(72) Inventors:
 • **DUCHATEAU, Alexander, Lucia, Leonardus**
 **B-3620 Lanaken (BE)**
 • **GEBHARD, Ronald**
 **NL-6271 JK Gulpen (NL)**
 • **BROXTERMAN, Quirinus, Bernardus**
 **NL-6151 JA Munstergeleen (NL)**

(74) Representative: **Breepoel, Peter M.**
**DSM Intellectual Property**
**Office Geleen**
**P.O. Box 9**
**6160 MA Geleen (NL)**

(56) References cited:
**US-A- 4 172 846**

 • **DATABASE WPI Derwent Publications Ltd., London, GB; AN 1986-337209 XP002236372 & SE 8 501 132 A (PERSTORP AB) cited in the application**
 • **WONJAE LEE: "Chromatographic separation of the enantiomers of amino acid esters as benzophenone imine derivatives" BULLETIN OF THE KOREAN CHEMICAL SOCIETY, vol. 19, no. 7, 1998, pages 715-717, XP002236371**

**Description**

The invention relates to a process for the preparation of an enantiomerically enriched Schiff base wherein an amine with formula 1

$$H_2N\text{-}R^1 \qquad (1)$$

is contacted with a carbonyl compound, with formula 2

$$R^2\text{-}C(O)\text{-}R^3 \qquad (2)$$

wherein the amine and/or the carbonyl compound is a chiral compound, to form a mixture of the enantiomers (or diastereomers where appropriate) of the corresponding Schiff base with formula 3

$$R^2\text{-}C(R^3)\text{=}N\text{-}R^1 \qquad (3)$$

wherein, if the amine is the chiral compound $R^1$ represents a chiral group chosen from an alkyl, (hetero)aryl, alkoxy, (hetero)aryloxy, (di)alkylamino, acylamino or (hetero)arylamino group, $R^2$ represents an (hetero)aryl group and $R^3$ represents H, if the carbonyl compound is the chiral compound $R^2$ and $R^3$ each independently represent H, an alkyl or (hetero)aryl group with the proviso that the carbonyl compound is chiral, and $R^1$ represents an (hetero)aryl group or an (hetero)aryl substituted C2-C10 alkyl group wherein the (hetero)aryl substituent is not in the $\alpha$-position relative to the imine-N, and if both the amine and the carbonyl compound are chiral compounds, $R^1$, $R^2$ and $R^3$ in combination may have the same meanings as given above for both the chiral amine and the chiral carbonyl compound situation, and the mixture of enantiomers of the Schiff base is subjected to preparative chromatography on a stationary phase whereby separation of the enantiomers of the Schiff base is obtained. The enantiomerically enriched Schiff bases obtained may subsequently be hydrolyzed to give, in case the amine is the chiral compound to be resolved, the corresponding enantiomerically enriched amine, or, in case the carbonyl compound is the chiral compound to be resolved, the enantiomerically enriched carbonyl compound.

The separation of alkanol amines using liquid chromatography via derivatization, particularly via derivatization into an oxazolidine, is described in SE-8501132-8. The separation of the enantiomers using this process proved to be rather bad.

Surprisingly it has been found that the process according to the invention can be advantageously used for the resolution of chiral amines as well as chiral carbonyl compounds, based on the common inventive concept that the resolution of the corresponding Schiff bases using preparative chromatography leads to a much better separation of the enantiomers than the separation obtained in the known process. This is the more surprising as it was to be expected that Schiff bases are more sensitive to racemisation.

In a preferred embodiment of the invention the undesired enantiomer of the Schiff base is subjected to racemisation. Subsequently the mixture of the enantiomers of the Schiff base obtained is subjected to the preparative chromatographic step according to the invention.

The Schiff base to be subjected to preparative chromatography may be a mixture of cis and trans isomers. Preferably the preparation of the Schiff base is performed such that preferentially one isomer (either cis or trans) is obtained. Most preferably the excess of such isomer with respect to the other is as high as possible.

The term "chiral compound" refers to compounds with either a chiral carbon atom, or a configurationally stable chiral heteroatom. Compounds where chirality is caused by restricted rotation or is due to the overall threedimensional shape, e.g. a helical shape, and suitable substituted adamantanes are also termed "chiral compounds".

The term "chiral center" refers to any structural feature of a molecule that gives rise to different enantiomers.

The term "alkyl" refers to an optionally substituted alkyl group with for instance 1-25, in particular 1-10 C-atoms, for example optionally asymmetrically substituted methyl, ethyl, propyl, isopropyl, butyl and octyl groups. Suitable substituents are for instance, halogens, hydroxy, C1-C6 alkenyl, C1-C6 alkynyl, C1-C6 alkoxy, thio, C1-C6 alkylthio, amino, C1-C6 alkylamino, C1-C6 acyloxy, C1-C6 acylthio, C1-C6 acylamino, nitro, cyano, carboxy, C1-C6 alkoxyacyl, acyl, (C1-C6 alkyl substituted) amino acyl, C3-C20 (hetero)aryl groups.

The term "aryl" refers to an optionally substituted aromatic hydrocarbon group, for instance a phenyl or naphtyl group with for example 5-25 C-atoms. Suitable substituent(s) are, for instance, alkyl groups, for instance C1-C6 alkyl, and the substituents described above in relation to alkyl groups.

The term "heteroaryl" refers to optionally substituted aromatic ring systems with for instance 3-20 C-atoms, for instance aromatic ring systems having in the ring(s) 3-10 C-atoms and at least one heteroatom, in particular O, N or S, for example furyl, thienyl, pyridinyl, indolyl and quinolyl. The ring(s) may be substituted, for instance with substituents mentioned above in relation to aryl groups.

The term "alkoxy" refers to an optionally substituted straight chain or branched chain alkoxy group with, for instance 1-25, in particular 1-10 C-atoms, in particular methoxy, ethoxy, propoxy, isopropoxy, butoxy, *tert*-butoxy and pentoxy. The alkoxy group may be substituted, for instance with substituents mentioned above for aryl groups.

Preferably the chiral center in the Schiff base is located at the $\alpha$- or $\beta$-position relative to the imine-N (in $R^1$, $R^2$ and/or $R^3$), most preferably at the $\alpha$-position. The groups $R^1$, $R^2$ and/or $R^3$ may contain functional groups that are inert in the imine forming and/or removal reaction or that are protected by suitable protecting groups.

In the resolution of chiral amines via Schiff base formation according to the invention a broad range of (non chiral) aldehydes can be used. Preferably a benzaldehyde with 0-5 substituents is used as the aldehyde. Suitable substituents are for example halogens, hydroxy, C1-C6 alkyl, C1-C6 alkoxy groups. Preferably easily accessible benzaldehydes with a good performance in the process of the invention are used, for example a benzaldehyde with 0, 1 or 2 substituents.

In the resolution of chiral amines preferably a non chiral aldehyde is used. If a mixture of the enantiomers of the aldehyde is used as a starting material 4 stereoisomers are formed. Therefore, if the aldehyde is chiral, the aldehyde is preferably used in enantiomerically pure form, for instance with an ee > 95%, preferably > 98%, more preferably > 99%. It will be clear, however, that if the racemic amine and carbonyl compound both are very cheap, it may also be cost effective to use both the amine and the aldehyde in racemic (or unresolved) form as starting materials in the process of the present invention.

By choosing a specific aldehyde in combination with the amine (to be resolved), it appeared possible to find Schiff bases with good solubility in the mixture to be separated. This good solubility contributes to a high production capacity which leads to a commercially attractive process.

In the resolution of carbonyl compounds via Schiff base formation according to the invention a broad range of (non chiral) amines $NH_2R^1$, wherein $R^1$ represents an (hetero)aryl group or an (hetero)aryl substituted C2-C10 alkyl group, can be used, provided that the (hetero)aryl substituent is not in the $\alpha$-position relative to the imine-N. Enantiomerically enriched carbonyl compounds that can be prepared with the process according to the invention are chiral carbonyl compounds with formula 2, wherein $R^2$ and $R^3$ each independently represent H, an alkyl group with for instance 1-20 C-atoms, an (hetero)aryl group with for instance 3-25 C-atoms. The process of the present invention is particularly suited for the resolution of aldehydes, the carbonyl compounds of formula 2 with $R^2$ or $R^3$ is H.

In the resolution of chiral carbonyl compounds preferably a non chiral amine is used. If a mixture of the enantiomers of the amine is used as a starting material 4 stereoisomers are formed. Therefore, if the amine is chiral, the amine is preferably used in enantiomerically pure form, for instance with an ee > 95%, preferably > 98%, more preferably > 99%. It will be clear, however, that if the racemic amine and carbonyl compound both are very cheap, it may also be cost effective to use both the amine and the carbonyl compound in racemic (or unresolved) form as starting materials in the process of the present invention.

By choosing a specific amine in combination with the carbonyl compound (to be resolved), it appeared possible to find Schiff bases with good solubility in the mixture to be separated. This good solubility contributes to a high production capacity which leads to a commercially attractive process.

The process for the preparation of an enantiomerically enriched Schiff base according to the invention is carried out by preparative chromatography on a chiral stationary phase.

The term "preparative chromatographic separation" relates to methods of separating mixtures of enantiomers or diastereomers which are dissolved in the mobile phase, of sufficient scale to isolate relevant quantities of the enantiomer or diastereomer desired. Such methods are known in the art. A suitable method for preparative chromatographic separation is, for instance, adsorption chromatography, e.g. column chromatography. Particularly preferred separation methods are those known as HPLC (high performance liquid chromatography), SFC (supercritical fluid chromatography), both in batch mode and in continuous mode, e.g. SMB (simulated moving bed chromatography). In the separation of enantiomers these methods involve the use of a chiral stationary phase. In case only 2 diastereomers need to be separated, of course, also an achiral stationary phase may be used.

As is well known by the skilled person the term "stationary phase" relates to a suitable inert carrier material on which an interacting agent is immobilized. The term "chiral stationary phase" relates to stationary phases in which the interacting agent is an enantiomerically enriched resolving agent, for instance immobilized by coating, by chemically binding or by insolubilizing via cross-linking on an inert carrier material. A suitable inert carrier material is preferably macroporous, e.g. crosslinked polystyrene, polyacrylamide, polyacrylate, alumina, kieselgur, quartz, kaolin, magnesium oxide or titanium dioxide. Silicagel is particularly preferred. Examples of stationary phases containing an enantiomerically enriched resolving agent are, for instance, phases based on either synthetic or naturally occurring chiral polymers, macrocyclic phases, ligand-exchange phases and Pirkle-type phases. Such chiral stationary phases are known and commercially available. Particularly preferred are polysaccharide phases, for instance Chiralcel OD® , Chiralcel OJ® , Chiralpak AD® and Chiralpak AS® (all Daicel).

The term "mobile phase" relates to a solvent or mixture of solvents in which the mixture of enantiomers to be separated is dissolved. Suitable solvents to be used in the preparative chromatographic process according to the invention are the solvents that are known to be used in analytical chromatography. In liquid chromatography as a rule non-polar, polar protic or aprotic solvents, or mixtures thereof are used. In supercritical chromatography preferably mixtures of carbon dioxide and polar protic solvents are used.

Suitable non polar solvents are for example hydrocarbons, for instance n-pentane, n-hexane and n-heptane.

Suitable polar protic or aprotic solvents are for example alcohols, in particular methanol, ethanol, 1-propanol, 2-propanol, 1-butanol, 2-butanol, isobutanol, tert butanol; ethers; esters, for instance ethylacetate; halogenated hydrocarbons and acetonitrile. The addition of small amounts of water, acid (for instance formic acid, acetic acid, trifluoroacetic acid) or base (for instance organic bases, e.g. triethylamine) for example less than 1 % (v/v) in the solvent may have advantageous effects.

In liquid chromatography, it is preferred to use lower, for instance C1-C3, alcohols or mixtures of these alcohols with hydrocarbons, for instance n-hexane or n-heptane. In supercritical chromatography mixtures of carbon dioxide and polar protic solvents, e.g. methanol, are preferred. The optimal solvent (combination) can be screened using methods known in the art. A different optimal solvent (combination) may be found when another stationary phase is used.

It appeared that the solubility of the Schiff base as a rule was higher than the parent compound, leading to higher production capacities. The process of the present invention, therefore can be performed at relatively high concentrations of the Schiff base in the mixture to be resolved, for instance at concentrations between 0.5-10% (w/v) of Schiff base in the mixture to be resolved. As a result it appeared possible to obtain a commercially attractive process for resolving chiral Schiff bases, chiral amines and chiral carbonyl compounds.

The present invention will now be described in detail with reference to the following examples that by no means limit the scope of the invention.

Materials used and definitions.

The carrier material of the HPLC columns (5 x 0.46 cm I.D. and 25 x 0.46 cm I.D.) consists of silicagel, granular size 10 $\mu$m, coated with amylose tris (3,5-dimethylphenylcarbamate) (CHIRALPAK AD® ), amylose tris ((S)-$\alpha$-methyl-benzylcarbamate (CHIRALPAK AS® ), cellulose tris (3,5-dimethylphenylcarbamate (CHIRALCEL OD® ) and cellulose tris (4-methylbenzoate) (CHIRALCEL OJ® ).

A Gilson 302 HPLC pump was used for solvent delivery and a Rheodyne 7010 valve for injection. Detection of the column effluent was carried out with an UV detector, Spectrasystem UV2000

The definitions of the terms used in the examples are as follows:

$$\text{Capacity factor } (k_n') = \frac{(\text{retention volume of peak number n}) - (\text{dead volume})}{(\text{dead volume})}$$

$$\text{Separation factor } (\alpha) = \frac{(\text{Capacity factor of more strongly retained isomer})}{(\text{Capacity factor of less strongly retained isomer})}$$

Example 1

Schiff base derivatives of chiral amines and benzaldehyde were chromatographed on a stationary phase of CHIRALPAK ® AD, CHIRALCEL® OD, CHIRALCEL® OJ and CHIRALPAK® AS using 5 x 0.46 cm I.D. columns at room temperature, at a flow-rate of 1 ml/min, utilizing a mixture of n-hexane and isopropanol (IPA) as the mobile phase. The percentage (v/v) of IPA used in the mobile phase is given in Table 1. Separation of the enantiomers was measured by UV absorption. The results are represented in Table 1.

Table 1: Separation of the enantiomers of Schiff base derivatives of chiral primary amines and benzaldehyde

| Benzaldehyde Schiff base derivative of | Chiralpak AD | | | Chiralcel OD | | | Chiralcel OJ | | | Chiralpak AS | | |
|---|---|---|---|---|---|---|---|---|---|---|---|---|
| | $K_1$ | α | IPA %(v/v) | $k_1$ | α | IPA %(v/v) | $k_1$ | α | IPA %(v/v) | $k_1$ | α | IPA %(v/v) |
| (structure) | 2.62 | 1.13 | 0.5 | >7 | -- | 5 | 2.98 | 1.71 | 20 | 0.58 | 1 | 20 |
| (structure) | 1.30 | 1.60 | 20 | 5.02 | 1.33 | 20 | 2.32 | 1.35 | 20 | 4.16 | 1 | 100 |
| (structure) | 0.90 | 1.60 | 10 | 2.88 | 2.15 | 10 | 2.00 | 1.10 | 5 | 1.28 | 2.14 | 10 |
| (structure) | 2.06 | 2.23 | 5 | 3.12 | 1.18 | 44 | 0.52 | 3.04 | 44 | 3.46 | 1 | 44 |
| (structure) | 1.48 | 1.50 | 36 | 5.44 | 1.17 | 36 | 2.78 | 1.56 | 36 | >7 | -- | 100 |
| (structure) | 2.54 | 1.39 | 10 | 4.66 | 1.43 | 20 | 2.70 | 1.89 | 60 | 3.20 | 1 | 40 |
| (structure) | 1.58 | 1.15 | 44 | 1.04 | 1.42 | 44 | 1.08 | 1.13 | 80 | 1.36 | 1 | 44 |
| (structure, erythro) | 1.42 | 1.20 | 0.1 | 0.86 | 1.95 | 5 | 1.78 | 1.26 | 44 | 1.00 | 1.58 | 0.1 |
| (structure) | 0.38 | 1 | 1 | 1.88 | 1.20 | 5 | 0.76 | 1 | 1.0 | 0.56 | 1 | 1.0 |

## Example 2

Schiff base derivatives of chiral amines and several ring-substituted benzaldehydes were chromatographed on a stationary phase of CHIRALPAK® AD using a 25 x 0.46 cm I.D. column at room temperature, at a flow-rate of 1 ml/min, utilizing a mixture of n-hexane and isopropanol (IPA; vol-% IPA in the mobile phase as indicated in the table) as the mobile phase. Separation of the enantiomers was measured by UV absorption. The results are represented in Table 2.

## Table 2: Separation of the enantiomers of Schiff base derivatives of chiral primary amines and several ring-substituted benzaldehydes (B)

| | 4-methoxy-B | | | 4-methyl-B | | | 3,4-dimethoxy-B | | | 4-chloro-B | | | 3-nitro-B | | | 2-hydroxy-B | | |
|---|---|---|---|---|---|---|---|---|---|---|---|---|---|---|---|---|---|---|
| | $k_1$ | α | IPA % (v/v) | $k_1$ | α | IPA % (v/v) | $k_1$ | α | IPA % (v/v) | $k_1$ | α | IPA % (v/v) | $k_1$ | α | IPA % (v/v) | $k_1$ | α | IPA % (v/v) |
| (structure) | 5.48 | 1.24 | 1,0 | 3.72 | 1.19 | 1.0 | > 11 | ---- | 1.0 | 4.60 | 1.11 | 1.0 | > 7 | --- | 1.0 | 10.28 | 1.02 | 1.0 |
| (structure) | 2.09 | 1.68 | 10 | 1.35 | 1.38 | 10 | 5.95 | 1.73 | 10 | 2.74 | 1.51 | 10 | 8.05 | 1.35 | 10 | 6.15 | 1.08 | 10 |
| (structure) | 1.89 | 1.61 | 10 | 1.12 | 1.55 | 10 | 3.70 | 1.39 | 10 | 1.29 | 1.71 | 10 | 3.43 | 1 | 10 | 2.03 | 1.55 | 10 |
| (structure) | 2.89 | 2.02 | 10 | 1.35 | 1.98 | 10 | 4.28 | 2.32 | 10 | 1.56 | 2.36 | 10 | 4.72 | 1.86 | 10 | 2.58 | 1.90 | 10 |
| (structure) | 1.30 | 1.58 | 44 | 2.82 | 1.50 | 14 | 1.76 | 1.51 | 44 | 1.22 | 1.46 | 44 | 2.32 | 1.28 | 44 | 1.48 | 1.32 | 44 |
| (structure) | 2.56 | 1.42 | 20 | 1,60 | 1.38 | 20 | > 11 | --- | 20 | 1.88 | 1.40 | 20 | 4.59 | 1.44 | 20 | 2.56 | 1.69 | 20 |
| (structure) erythro | 1.80 | 1.12 | 44 | --- | --- | --- | --- | --- | --- | 1.57 | 1.14 | 44 | 2.76 | 1.21 | 44 | 2.04 | 1.17 | 44 |
| (structure) | 4.75 | 1 | 0.1 | 2.08 | 1.26 | 0.1 | > 11 | --- | 0.1 | 2.14 | 1.27 | 0.1 | > 11 | --- | 0.1 | 5.87 | 1 | 0.1 |
| (structure) | --- | --- | --- | 0.54 | 1 | 1.0 | 3.63 | 1.03 | 1.0 | 0.52 | 1 | 1.0 | 1.18 | 1 | 1.0 | 0.82 | 1 | 1.0 |

EP 1 565 427 B1

Example 3

Schiff base derivatives of chiral aldehydes and amines were chromatographed on a stationary phase of CHIRALPAK® AD and CHIRALCEL® OD using 5 x 0.46 cm I.D. columns at room temperature, at a flow-rate of 1 ml/min, utilizing a mixture of n-hexane and isopropanol (IPA; vol-% IPA in the mobile phase as indicated in the table) as the mobile phase. Separation of the enantiomers was measured by UV absorption. For chiral aldehyde (I), 2-fenylethylamine was used for Schiff base formation. For chiral aldehyde (II), p-anisidine was used for Schiff base formation. The results are represented in Table 3.

### Table 3

## Separation of the enantiomers of Schiff base derivatives of chiral aldehydes and amines

| Schiff base[1] derivative of | Chiralcel OD | | | Chiralpak AD | | |
|---|---|---|---|---|---|---|
| | $k_1$ | $\alpha$ | IPA %(v/v) | $k_1$ | $\alpha$ | IPA %(v/v) |
| (I) | 1.45 | 1.27 | 3 | | | |
| (II) | | | | 4.66 | 1.23 | 10 |

Example 4

Determination of productivity of a SMB process for the benzaldehyde Schiff base of 2-amino-2-*tert*.butylacetamide (dl-*tert*. leucine amide)

For the benzaldehyde Schiff base of 2-amino-2-*tert*.butylacetamide, the adsorption isotherms of the enantiomers have been determined using a perturbation method (as described by C. Heuer, E.Küsters, T.Plattner and A.Seidel-Morgenstern, J.Chromatogr.A.,vol.827 (1998) pp.175-191). The column used was a 5 x 0.46 cm I.D. Chiralpak AD from Daicel. 2-Propanol was used as mobile phase at a flow-rate of 1.0 ml/min. Injection volume was 20 μl. Residence times of both enantiomers were measured at 14 concentration levels (between 4 and 46 g racemate /l). The experiment has been performed at room temperature.

Several types of adsorption isotherms have been examined for the description of the data. Best fit was found for the modified Langmuir isotherm. Using the parameters for the modified Langmuir isotherm, the TMB/SMB operation region was calculated according to the equilibrium theory. The feed concentration was fixed at 46 g/l.

The performance of various TMB and SMB configurations with a set of flow-rates was simulated using an in-house developed Aspen Custom Modeler model (TMB) and Aspen Chromatography (SMB).

For a six column configuration, the production rate is 1 kg (2-amino-2-*tert*.butylacetamide) enantiomer per kg stationary phase per day.

**Claims**

**1.** Process for the preparation of an enantiomerically enriched Schiff base wherein an amine with formula 1

$$H_2N\text{-}R^1, \qquad (1)$$

is contacted with a carbonyl compound, with formula 2

$$R^2\text{-C(O)-}R^3 \qquad (2)$$

wherein the amine and/or the carbonyl compound is a chiral compound, to form a mixture of the enantiomers or diastereomers of the corresponding Schiff base with formula 3

$$R^2\text{-C}(R^3)\text{=N-}R^1 \qquad (3)$$

wherein, if the amine is the chiral compound $R^1$ represents a chiral group chosen from an alkyl, (hetero)aryl, alkoxy, (hetero)aryloxy, (di)alkylamino, acylamino or (hetero)arylamino group, $R^2$ represents an (hetero)aryl group and $R^3$ represents H, if the carbonyl compound is the chiral compound $R^2$ and $R^3$ each independently represent H, an alkyl, or (hetero)aryl, group with the proviso that the carbonyl compound is chiral and $R^1$ represents an (hetero)aryl group or an (hetero)aryl substituted C2-C10 alkyl group wherein the (hetero)aryl substituent is not in the α-position relative to the imine-N, and if both the amine and the carbonyl compound are chiral compounds, $R^1$, $R^2$ and $R^3$ in combination may have the same meanings as given above for both the chiral amine and the chiral carbonyl compound situation, and the mixture of enantiomers of the Schiff base is subjected to preparative chromatography on a stationary phase whereby separation of the enantiomers of the Schiff base is obtained.

2. Process according to claim 1, wherein a mixture of diastereomers of the Schiff base is subjected to preparative chromatography.

3. Process according to claims 1 or 2, wherein a chiral stationary phase is used.

4. Process according to any one of claims 1 - 3, wherein the preparative chromatography used is Simulated Moving Bed chromatography.

5. Process according to any one of claims 1-4, wherein the chiral center in the Schiff base is at the α- or β-position relative to the imine-N, most preferably at the α-position.

6. Process according to any one of claims 1-5, wherein the amine is the chiral compound and the carbonyl compound is achiral.

7. Process according to any one of claims 1-6, wherein the amine is chiral and which process further comprises hydrolyzing the enantiomerically enriched Schiff base to form the corresponding enantiomerically enriched amine.

8. Process according to claim 6 or 7, wherein the carbonyl compound is a benzaldehyde.

9. Process according to any one of claims 1-5, wherein the carbonyl compound is the chiral compound.

10. Process according to claim 10, wherein the amine is achiral.

11. Process according to claim 9 or 10, which process further comprises hydrolyzing the enantiomerically enriched Schiff base to form the corresponding enantiomerically enriched carbonyl compound.

12. Process according to claim 9, wherein the carbonyl compound is an aldehyde.

13. Process according to any one of claims 1-12, wherein the concentration of Schiff base in the mixture to be resolved is between 0.5 and 10 % by (w/v).

14. Process according to any one of claims 1-13, wherein preparative liquid chromatography is used and wherein the mixture of the enantiomers of the Schiff base is dissolved in an alcohol, a hydrocarbon or any mixture thereof.

15. Process according to any one of claims 1-13, wherein preparative super- critical chromatography is used and wherein the mixture of enantiomers of the Schiff base is dissolved in a mixture of carbon dioxide and a polar protic solvent.

16. Process according to any one of claims 1-15, wherein the undesired enantiomer of the Schiff base is subjected to racemisation and subsequently the mixture of enantiomers obtained is recycled to the preparative chromatographic step.

**EP 1 565 427 B1**

**Patentansprüche**

1. Verfahren zur Herstellung einer enantiomer angereicherten Schiffschen Base, wobei ein Amin der Formel 1

$$H_2N\text{-}R^1 \qquad (1)$$

mit einer Carbonylverbindung der Formel 2

$$R^2\text{-}C(O)\text{-}R^3, \qquad (2)$$

wobei das Amin und/oder die Carbonylverbindung eine chirale Verbindung ist, unter Bildung einer Mischung der Enantiomere oder Diastereomere der entsprechenden Schiffschen Base der Formel 3

$$R^2\text{-}C(R^3)\text{=}N\text{-}R^1 \qquad (3),$$

in Kontakt gebracht wird, wobei, wenn das Amin die chirale Verbindung ist, $R^1$ eine chirale Gruppe darstellt, die unter einer Alkyl-, (Hetero)aryl-, Alkoxy-, (Hetero)aryloxy-, (Di)alkylamino-, Acylamino- oder (Hetero)arylaminogruppe ausgewählt ist, $R^2$ eine (Hetero)arylgruppe und $R^3$ H darstellt, wenn die Carbonylverbindung die chirale Verbindung ist, $R^2$ und $R^3$ jeweils unabhängig H, eine Alkyl- oder (Hetero)arylgruppe darstellen unter der Voraussetzung, dass die Carbonylverbindung chiral ist und $R^1$ eine (Hetero)arylgruppe oder eine durch (Hetero)aryl substituierte C2-C10-Alkylgruppe darstellt, wobei der (Hetero)arylsubstituent sich nicht in der $\alpha$-Stellung mit Bezug auf das Imin-N befindet und, wenn sowohl das Amin als auch die Carbonylverbindung chirale Verbindungen sind, $R^1$, $R^2$ und $R^3$ in Kombination die gleiche Bedeutung haben können wie oben sowohl für die Situation des chiralen Amins als auch die Situation der chiralen Carbonylverbindung angegeben und die Mischung von Enantiomeren der Schiffschen Base der präparativen Chromatographie an einer stationären Phase unterworfen wird, wobei die Trennung der Enantiomere der Schiffschen Base erzielt wird.

2. Verfahren nach Anspruch 1, wobei eine Mischung von Diastereomeren der Schiffschen Base der präparativen Chromatographie unterworfen wird.

3. Verfahren nach Anspruch 1 oder 2, wobei eine chirale stationäre Phase verwendet wird.

4. Verfahren nach einem der Ansprüche 1 - 3, wobei die präparative Chromatographie bei der simulierten Chromatographie im Fließbett verwendet wird.

5. Verfahren nach einem der Ansprüche 1 - 4, wobei das Chiralitätszentrum in der Schiffschen Base sich mit Bezug auf das Imin-N in der $\alpha$- oder $\beta$-Stellung, am bevorzugtesten in der $\alpha$-Stellung, befindet.

6. Verfahren nach einem der Ansprüche 1 - 5, wobei das Amin die chirale Verbindung und die Carbonylverbindung achiral ist.

7. Verfahren nach einem der Ansprüche 1 - 6, wobei das Amin chiral ist und das Verfahren des Weiteren das Hydrolysieren der enantiomer angereicherten Schiffschen Base unter Bildung des entsprechenden enantiomer angereicherten Amins umfasst.

8. Verfahren nach Anspruch 6 oder 7, wobei die Carbonylverbindung ein Benzaldehyd ist.

9. Verfahren nach einem der Ansprüche 1 - 5, wobei die Carbonylverbindung die chirale Verbindung ist.

10. Verfahren nach Anspruch 10, wobei das Amin achiral ist.

11. Verfahren nach Anspruch 9 oder 10, welches des Weiteren das Hydrolysieren der enantiomer angereicherten Schiffschen Base unter Bildung der entsprechenden enantiomer angereicherten Carbonylverbindung umfasst.

12. Verfahren nach Anspruch 9, wobei die Carbonylverbindung ein Aldehyd ist.

13. Verfahren nach einem der Ansprüche 1 - 12, wobei die Konzentration an Schiffscher Base in der aufzulösenden Mischung zwischen 0,5 und 10 (Gew.-/Vol.-) % beträgt.

**14.** Verfahren nach einem der Ansprüche 1 - 13, wobei präparative Flüssigchromatographie angewendet X wird und wobei die Mischung der Enantiomere der Schiffschen Base in einem Alkohol, einem Kohlenwasserstoff oder einer Mischung derselben gelöst wird.

**15.** Verfahren nach einem der Ansprüche 1 - 13, wobei präparative superkritische Chromatographie X angewendet wird und wobei die Mischung von Enantiomeren der Schiffschen Base in einer Mischung von Kohlendioxid und einem polaren protischen Lösungsmittel gelöst wird.

**16.** Verfahren nach einem der Ansprüche 1 - 15, wobei das unerwünschte Enantiomer der Schiffschen Base einer Racemisierung unterworfen wird und die erhaltene Mischung von Enantiomeren daraufhin zur präparativen chromatographischen Stufe zurückgeführt wird.

**Revendications**

**1.** Procédé de préparation d'une base de Schiff enrichie en énantiomère dans lequel on met en contact une amine de formule 1

$$H_2N\text{-}R^1 \qquad (1)$$

avec un composé carbonyle, de formule 2

$$R^2\text{-}C(O)\text{-}R^3 \qquad (2)$$

dans laquelle l'amine et/ou le composé carbonyle est un composé chiral, pour former un mélange des énantiomères ou des diastéréoisomères de la base de Schiff correspondante de formule 3

$$R^2\text{-}C(R^3)=N\text{-}R^1 \qquad (3)$$

dans laquelle, si l'amine est le composé chiral, $R^1$ représente un groupe chiral choisi parmi un groupe alkyle, (hétéro)aryle, alcoxy, (hétéro)aryloxy, (di)alkylamino, acylamino ou (hétéro)arylamino, $R^2$ représente un groupe (hétéro)aryle et $R^3$ représente H, si le composé carbonyle est le composé chiral, $R^2$ et $R^3$ représentent chacun indépendamment H ou un groupe alkyle ou (hétéro)aryle, sous réserve que le composé carbonyle soit chiral et que $R^1$ représente un groupe (hétéro)aryle ou alkyle en $C_2\text{-}C_{10}$ substitué par un groupe (hétéro)aryle dans lequel le substituant (hétéro)aryle n'est pas en position $\alpha$ par rapport à l'atome de N du groupe imine, et si à la fois l'amine et le composé carbonyle sont des composés chiraux, $R^1$, $R^2$ et $R^3$ en combinaison peuvent avoir les mêmes définitions que celles indiquées ci-dessus à la fois pour le cas de l'amine chirale et pour le cas du composé carbonyle chiral, et le mélange d'énantiomères de la base de Schiff est soumis à une chromatographie préparative sur une phase stationnaire ce qui permet d'obtenir une séparation des énantiomères de la base de Schiff.

**2.** Procédé selon la revendication 1, dans lequel un mélange de diastéréoisomères de la base de Schiff est soumis à une chromatographie préparative.

**3.** Procédé selon les revendications 1 ou 2, dans lequel on utilise une phase stationnaire chirale.

**4.** Procédé selon l'une quelconque des revendications 1 - 3, dans lequel la chromatographie préparative utilisée est une chromatographie en lit mobile simulé.

**5.** Procédé selon l'une quelconque des revendications 1-4, dans lequel le centre chiral dans la base de Schiff est en position $\alpha$ ou $\beta$ par rapport à l'atome de N de l'imine, tout particulièrement en position $\alpha$.

**6.** Procédé selon l'une quelconque des revendications 1-5, dans lequel l'amine est le composé chiral et le composé carbonyle est achiral.

**7.** Procédé selon l'une quelconque des revendications 1-6, dans lequel l'amine est chirale et ce procédé comprend en outre l'hydrolyse de la base de Schiff enrichie en énantiomère pour former l'amine enrichie en énantiomère correspondante.

**8.** Procédé selon la revendication 6 ou 7, dans lequel le composé carbonyle est un benzaldéhyde.

**9.** Procédé selon l'une quelconque des revendications 1-5, dans lequel le composé carbonyle est le composé chiral.

**10.** Procédé selon la revendication 10, dans lequel l'amine est achirale.

**11.** Procédé selon la revendication 9 ou 10, ce procédé comprenant en outre l'hydrolyse de la base de Schiff enrichie en énantiomère pour former le composé carbonyle enrichi en énantiomère correspondant.

**12.** Procédé selon la revendication 9, dans lequel le composé carbonyle est un aldéhyde.

**13.** Procédé selon l'une quelconque des revendications 1-12, dans lequel la concentration de base de Schiff dans le mélange à résoudre est comprise entre 0,5 et 10% en poids/volume.

**14.** Procédé selon l'une quelconque des revendications 1-13, dans lequel une chromatographie liquide préparative est employée et dans lequel le mélange des énantiomères de la base de Schiff est dissous dans un alcool, un hydrocarbure ou tout mélange de ceux-ci.

**15.** Procédé selon l'une quelconque des revendications 1-13, dans lequel une chromatographie préparative super-critique est employée et dans lequel le mélange d'énantiomères de la base de Schiff est dissous dans un mélange de dioxyde de carbone et d'un solvant protique polaire.

**16.** Procédé selon l'une quelconque des revendications 1-15, dans lequel l'énantiomère indésirable de la base de Schiff est soumis à une racémisation et ensuite le mélange d'énantiomères obtenu est recyclé vers létape de chromato-graphie préparative.